(19) Europäisches Patentamt
European Patent Office
Office européen des brevets

(11) EP 0 834 313 A1

(12) **EUROPEAN PATENT APPLICATION**

(43) Date of publication:
**08.04.1998 Bulletin 1998/15**

(51) Int Cl.[6]: **A61K 31/40**

(21) Application number: **97306716.8**

(22) Date of filing: **01.09.1997**

(84) Designated Contracting States:
**AT BE CH DE DK ES FI FR GB GR IE IT LI LU MC NL PT SE**
Designated Extension States:
**AL LT LV RO SI**

(30) Priority: **30.08.1996 US 24869 P**
**26.08.1997 US**

(71) Applicant: **ELI LILLY AND COMPANY**
**Indianapolis, Indiana 46285 (US)**

(72) Inventors:
- **Jirousek, Michael R.**
  **Indianapolise, IN 46236 (US)**
- **Ways, Douglas K.**
  **Indianapolis, IN 46205 (US)**
- **Stramm, Lawrence E.**
  **Indianapolis, IN 46256 (US)**

(74) Representative: **Dean, John Paul et al**
**Withers & Rogers**
**4 Dyer's Buildings**
**Holborn**
**London EC1N 2JT (GB)**

(54) **Therapeutic treatment for central nervous system diseases associated with HIV infection**

(57) A method for treating central nervous system associated with HIV infection is disclosed, particularly using the isozyme selective PKC inhibitor, (S)-3,4-[N, N'-1,1'-((2''-ethoxy)-3'''(O)-4'''-(N,N-dimethylamino)-butane)-bis-(3,3'-indolyl)]-1(H)-pyrrole-2,5-dione hydrochloride salt.

EP 0 834 313 A1

## Description

This application claims the priority benefits of the U.S. Provisional application Serial No. 60/024,869 filed August 30, 1996.

## BACKGROUND OF THE INVENTION

1. Field of the Invention

The present invention is broadly directed to the use of a particular class of isozyme selective Protein Kinase C (PKC) inhibitors for treating central nervous system (CNS) diseases associated with HIV infection.

2. Description of Related Art

The HIV epidemic continues to grow at a rapid rate, and the clinical manifestations associated with this viral infection present increasingly more complex medical and socioeconomic problems. Acute HIV infection leads to a period of rapid viral replication, followed by viremia that results in infection of 1% or more of circulating T lymphocytes, the primary target of the virus. Viremia is transient, however, because the cells injected with HIV are removed from circulation by an effective host immune response that results in a 10- to 100-fold decrease in the HIV-infected T cells. Unfortunately, no effective therapy yet exists for preventing viral activation after exposure. Thus, although the initial host response is effective in reducing and controlling HIV-infected cell numbers, it is not sufficient to prevent the postintegration latent or low-level-persistent (LLP) asymptomatic infections of host reservoir cells, such as circulating CD4+ T lymphocytes and monocyte/macrophages. Thus, the ultimate pathogenic effects of HIV are not prevented and after induction from the latent or LLP state, acquired immune deficiency syndrome (AIDS) develops.

Central nervous system (CNS) disease is a prominent feature of HIV infected patients manifesting AIDS symptamology. Symptoms related to this CNS affliction include paralysis, dementia and death. While HIV associated CNS disease occurs in the setting of HIV infection, the etiology of the illness is likely due to the host response to the virus rather than to a direct viral cytolytic effect.

No cure has yet been found for HIV infection and CNS diseases associated therewith. Current treatments attempt to retard the progress of the disease or relieve its symptoms. While drugs have been used or proposed for treatment of HIV infection, including the recent introduction of several HIV protease inhibitors none have yet been demonstrated to be completely effective. In particular, no therapeutic agent has been offered to specifically treat CNS diseases associated with HIV infection. Therefore, there remains a need in the art to develop therapeutic agents to treat CNS diseases associated with HIV infection.

## SUMMARY OF INVENTION

It is an object of the invention to provide a method for treating central nervous system diseases associated with human immunodeficiency virus infection.

These and other objects of the invention are provided by one or more of the embodiments described below.

One embodiment of the invention provides a method for treating central nervous system diseases associated with human immunodeficiency viral infection which comprises administering to a patient in need of such treatment a therapeutically effective amount of a protein kinase C inhibitor.

The present invention thus provides the art with compounds effective in treating HIV associated CNS diseases.

## DETAILED DESCRIPTION OF THE INVENTION

It is a discovery of the present invention that a particular class of protein kinase C inhibitors, *i.e.*, inhibitors of the β isozyme of protein kinase C, and especially β isozyme selective inhibitors of PKC, has therapeutic effects on CNS diseases associated with HIV infection and specifically retards the effect that gp120 exerts on the HIV infected patient.

gp120 is a product of the HIV genome that is shed into the extracellular space from HIV infected cells. gp120 can induce both *in vitro* and *in vivo* neurotoxicity (Gendilman et al., 1994, J. Leukocyte Biol. 56:389-398; Crow et al., 1994, J. Leukocyte Biol, 56:215-217; Rosenberg Z and Fauci A, 1990. Immunol. Today 11: 176-180; Mosier D and Sieburg H, 1994. Immunol. Today 15: 332-339). gp120 positive astroglial cells, especially reactive astrocytosis or alternatively termed reactive gliosis, have been implicated in the CNS damage that leads to the CNS manifestations seen in HIV infected patients. It has been demonstrated that non HIV infected transgenic mice with targeted overexpression of gp 120 to astroglial cells display neuronal and glial damage that closely resembles what is seen in the brains of HIV infected patients exhibiting CNS symptamotology (Toggas et al., 1994. Nature 367: 188-193).

gp120 is known to activate PKC. PKC activity is up modulated in gp120 positive HIV-1 transfected cells, in the CNS of gp120 transgenic mice, and in the specimens from the brains of HIV infected patients. It is known that the presence of gp120 induces a prominent elevation of steady state glial fibrillary acidic protein (GFAP) mRNA levels in gp120 transgenic mice correlating with the activation of PKC (Wyss-Coray, *et al., J. Clin. Invest.*, 97(3):789-798 (1996)). GFAP causes astrocytosis, a condition closely related to CNS disease. The up-regulation of GFAP is involved in neuronal damage and promiment reactive astrocytosis of HIV-associated central nervous system (CNS) disease. The gp120 effects are diminished by PKC inhibitors but not by inhibitors of protein kinase A. (Wyss-Coray, *et al, J. Clin. Invest.,* 97(3);789-798 (1996))

Though not wishing to be limited to any technical explanation, applicant(s) believe that HIV related CNS neurotoxicity is due to gp120 induced PKC activation and reactive gliosis. The PKC pathway is a necessary component in the reactive glosis that occurs in patients with HIV related CNS diseases. The ability of PKC inhibitors to block gp120 induced astroglial cell activation demonstrates that a therapy specifically interfering with the PKC pathway could block the reactive astroglisosis and its concomitant neurotoxicity which leads to the clinical symptomology associated with HIV related CNS disease. Therefore, the present invention proposes PKC inhibitor compounds, exhibiting selectively for the β isozyme, for use therapeutically to retard or halt progression of CNS disorders and decrease the paralysis, dementis and death associated with HIV related CNS complications.

The method of this invention preferably utilizes those protein kinase C inhibitors that effectively inhibit the β isozyme. One suitable group of compounds are generally described in the prior art as bis-indolylmaleimides or macrocyclic bis-indolylmaleimides. Bis-indolylmaleimides well recognized in the prior art include those compounds described in U.S. Patents 5,621,098, 5,552,396, 5,545,636, 5,481,003, 5,491,242, and 5,057,614, all incorporated by reference herein. Macrocyclic bis-indolylmaleimides are particularly represented by the compounds of formula I. These compounds, and methods for their preparation, have been disclosed in U.S. Patent 5,552,396, which is incorporated herein by reference. These compounds are administered in a therapeutically effective amount to a human treat CNS diseases associated with HIV infection, especially to inhibit the gp120 effects in HIV infected patients. These compounds can also be administered to patients at risk of the disease conditions mentioned above as prophylactics.

One preferred class of compounds for use in the method of the invention has the formula (I):

(I)

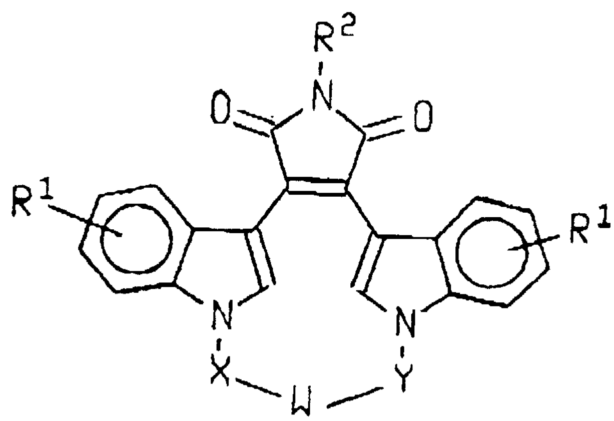

wherein:

W is -O-, -S-, -SO-, $-SO_2-$, -CO-, $C_2$-$C_6$, alkylene, substituted alkylene, $C_2$-$C_6$ alkenylene, -aryl-, $-aryl(CH_2)_mO-$, -heterocycle-, $-heterocycle-(CH_2)_mO-$, -fused bicyclic-, $-fused\ bicyclic-(CH_2)_mO-$, $-NR^3-$, $-NOR^3-$, -CONH-, or -NH-CO-;

X and Y are independently $C_1$-$C_4$ alkylene, substituted alkylene, or together X, Y, and W combine to form $-(CH_2)_m$-AA-;

$R^1$s are hydrogen or up to four optional substituents independently selected from halo, $C_1$-$C_4$ alkyl, hydroxy, $C_1$-$C_4$ alkoxy, haloalkyl, nitro, $-NR^4R^5$, or $-NHCO(C_1$-$C_4$ alkyl);

$R^2$ is hydrogen, $CH_3CO$-, $-NH_2$, or hydroxy;

$R^3$ is hydrogen, $-(CH_2)_m$aryl, $-C_1$-$C_4$ alkyl, $-COO(C_1$-$C_4$ alkyl), $-CONR^4R^5$, $-(C{=}NH)NH_2$, $-SO(C_1$-$C_4$ alkyl), $-SO_2(NR^4R^5)$, or $-SO_2$ ($C_1$-$C_4$ alkyl);

$R^4$ and $R^5$ are independently hydrogen, $C_1$-$C_4$ alkyl, phenyl, benzyl, or combine with the nitrogen to which they are bonded to form a saturated or unsaturated 5 or 6 member ring;

AA is an amino acid residue;

m is independently 0, 1, 2, or 3; and

n is independently 2, 3, 4, or 5,

or a pharmaceutically acceptable salt, prodrug or ester thereof.

A more preferred class of compounds for use in this invention is represented by formula I wherein the moieties -X-W-Y- contain 4 to 8 atoms, which may be substituted or unsubstituted. Most preferably, the moieties -X-W-Y- contain 6 atoms.

Other preferred compounds for use in the method of this invention are those compounds of formula I wherein $R^1$ and $R^2$ are hydrogen; and W is a substituted alkylene, -O-, S-, -CONH-, -NHCO- or $-NR^3-$. Particularly preferred compounds for use in the invention are compounds of the formula Ia:

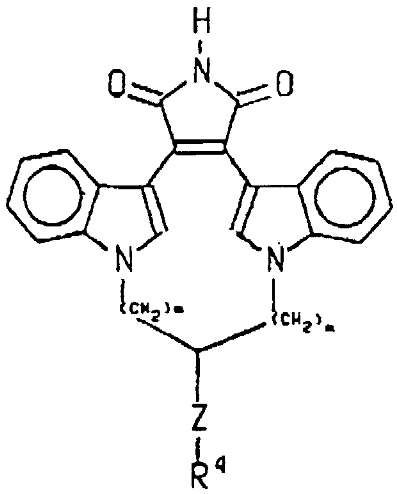

wherein Z is $-(CH_2)_p-$ or $-(CH_2)_p-O-(CH_2)_p-$; $R^4$ is hydroxy, -SH, $C_1$-$C_4$ alkyl, $(CH_2)_m$aryl, -NH(aryl), $-N(CH_3)(CF_3)$, $-NH(CF_3)$, or $-NR^5R^6$; $R^5$ is hydrogen or $C_1$-$C_6$ alkyl; $R^6$ is hydrogen, $C_3$-$C_4$ alkyl or benzyl; p is 0, 1, or 2; and m is independently 2 or 3, or a pharmaceutically acceptable salt, podrug or ester thereof. Most preferred compounds of the formula Ia are those wherein Z is $CH_2$; and $R^4$ is $-NH_2$, $-NH(CF_3)$, or $-N(CH_3)_2$, or a pharmaceutically acceptable salt, prodrug or ester thereof.

Other preferred compounds for use in the method of the present invention are compounds wherein W in formula I is -O-, Y is a substituted alkylene, and X is an alkylene. These preferred compounds are represented by formula Ib:

(Ib)

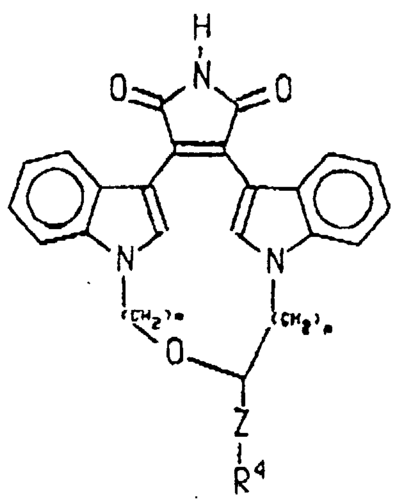

wherein Z is $-(CH_2)_p-$; $R^4$ is $-NR^5R^6$, $-NH(CF_3)$, or $-N(CH_3)(CF_3)$; $R^5$ and $R^6$ are independently H or $C_1$-$C_4$ alkyl; p is 0, 1, or 2; and m is independently 2 or 3, or a pharmaceutically acceptable salt, prodrug or ester thereof. Most preferred compounds of formula Ib are those wherein p is 1; and $R^5$ and $R^6$ are methyl.

Because they contain a basic moiety, the compounds of formulae I, Ia, and Ib can also exist as pharmaceutically

acceptable acid addition salts. Acids commonly employed to form such salts include inorganic acids such as hydrochloric, hydrobromic, hydroiodic, sulfuric and phosphoric acid, as well or organic acids such as para-toluenesulfonic, methanesulfonic, oxalic, para-bromophenylsulfonic, carbonic, succinic, citric, benzoic, acetic acid, and related inorganic and organic acids. Such pharmaceutically acceptable salts thus include sulfate, pyrosulfate, bisulfate, sulfite, bisulfite, phosphate, mono-hydrogenphosphate, dihydrogenphosphate, metaphosphate, pyrophosphate, chloride, bromide, iodide, acetate, propionate, decanoate, caprylate, acrylate, formate, isobutyrate, heptanoate, propiolate, oxalate, malonate, succinate, suberate, sebacate, fumarate, maleate, 2-butyne-1,4-dioate, 3-hexyne-2, 5-dioate, benzoate, chlorobenzoate, hydroxybenzoate, methoxybenzoate, phthalate, xylenesulfonate, phenylacetate, phenylpropionate, phenylbutyrate, citrate, lacate, hippurate, β-hydroxybutyrate, glycolate, maleate, tartrate, methanesulfonate, propanesulfonate, naphthalene-1-sulfonate, naphthalene-2-sulfonate, mandelate and the like. Particularly the hydrochloric and mesylate salts are used.

In addition to pharmaceutically-acceptable salts, other salts also can exist. They may serve as intermediates in the purification of the compounds, in the preparation of other salts, or in the identification and characterization of the compounds or intermediates.

The pharmaceutically acceptable salts of compounds of formulae I, Ia, and Ib can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent.

It is recognized that various stereoisometric forms of the compounds of formulae I, Ia, and Ib may exist; for example, W may contain a chiral carbon atom in the substituted alkylene moiety. The compounds are normally prepared as racemates and can conveniently be used as such. Alternatively, both individual enantiomers can be isolated or synthesized by conventional techniques if so desired. Such racemates and individual enantiomers and mixtures thereof form part of the compounds used in the methods of the present invention.

The compounds utilized in this invention also encompass the pharmaceutically. acceptable prodrugs of the compounds of formulae I, Ia, and Ib. A prodrug is a drug which has been chemically modified and may be biologicaliy inactive at its site of action, but which may be degraded or modified by one or more enzymatic or other *in vivo* processes to the parent bioactive form. This prodrug likely may have a different pharmacokinetic profile than the parent, enabling easier absorption across the mucosal epithelium, better salt formation or solubility, and/or improved systemic stability (an increase in plasma half-life, for example). Typically, such chemical modifications include the following:

1) ester or amide derivatives which may be cleaved by esterases or lipases;
2) peptides which may be recognized by specific or nonspecific proteases; or
3) derivatives that accumulate at a site of action through membrane selection of a prodrug form or a modified prodrug form; or any combination of 1 to 3, supra. Conventional procedures for the selection and preparation of suitable prodrug derivatives are described, for example, in H. Bundgaard, Design of Prodrugs, (1985).

The synthesis of various bis-indole-N-maleimide derivatives is described in Davis *et al.* U.S. Patent 5,057,614 and the synthesis of the preferred compounds suitable for use in this invention are described in the previously identified U.S. Patents 5,552,396 and in Faul *et al.* EP publication 0 657 411 A1, all of which are incorporated herein by reference.

One particularly preferred protein kinase -β inhibitor for use in the method of this invention is the compound described in Example 5g ((S)-3,4-[N,N'-1,1'-((2"-ethoxy)-3"'(O)-4"'-(N,N-dimethylamino)-butane)-bis-(3,3'-indolyl)]-1(H)-pyrrole-2,5-dione Hydrochloride Salt) of the aforementioned U.S. Patent 5,552,396. This compound is a potent protein kinase C inhibitor. It is selective to protein kinase C over other kinases and is highly isozyme-selective, i.e., it is selective for the beta-1 and beta -2 isozymes. Other salts of this compound also would be favored, especially the mesylate salts.

A preferred mesylate salt can be prepared by reacting a compound of the formula II:

(II)

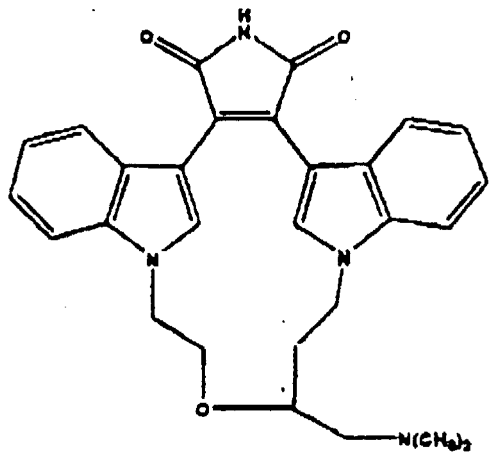

with methanesulfonic acid in a non-reactive organic solvent, preferably an organic/water mixture, and most preferably water-acetone. Other solvents such as methanol, acetone, ethylacetate and mixtures thereof are operable. The ratio of solvent to water is not critical and generally determined by the solubility of the reagents. Preferred solvent to water ratios are generally from 0.1:1 to 100:1 solvent to water by volume. Preferably, the ratio is 1:1 to 20:1 and most preferably 5:1 to 10:1. The optimal ratio is dependent on the solvent selected and is preferably acetone at a 9:1 solvent to water ratio.

The reaction usually involves approximately equimolar amounts of the two reagents, although other ratios, especially those wherein the methanesulfonic acid is in excess, are operative. The rate of addition of methanesulfonic acid is not critical to the reaction and may be added rapidly (<5 minutes) or slowly over 6 or more hours. The reaction is carried out at temperatures ranging from 0°C to reflux. The reaction mixture is stirred until formation of the salt is complete, as determined by x-ray powder diffraction and can take from 5 minutes to 12 hours.

The salts of the present invention are preferably and readily prepared as a crystalline form. The trihydrate form of the salt may be readily converted to the monohydrate upon drying or exposure to 20-60% relative humidity. The salt is substantially crystalline demonstrating a defined melting point, birefringence, and an x-ray diffraction pattern. Generally, the crystals have less than 10% amorphous solid and preferably less than 5% and most preferably less than 1% amorphous solid.

The mesylate salt is isolated by filtration or other separation techniques appreciated in the art, directly from the reaction mixture in yields ranging from 50% to 100%. Recrystallization and other purification techniques known in the art may be used to purify the salt further if desired.

One skilled in the art will recognize that a therapeutically effective amount of the protein kinase C inhibitor of the present invention is the amount sufficient to ameliorate the clinical symptomology of CNS diseases associated with HIV infection or the amount sufficient to inhibit gp120 induced CNS disorders. It is well within the ability of a person skilled in the art to measure the gp120 neurotoxicity including but not limited to neuronal damages associated with CNS diseases. The amount administered varies *inter alia*, depending upon the concentration of the compound in the therapeutic formulation, and the body weight of the patient. Generally, an amount of protein kinase C inhibitor to be administered as a therapeutic agent for HIV associated CNS disease will be determined on a case by case basis by the attending physician. As a guideline, the degree of infection, the strength of the immune system, the body weight and age of the patient will be considered when setting an appropriate dose.

Generally, a suitable dose is one that results in a concentration of the protein kinase C inhibitor at the treatment site in the range of 0.5 nM to 200 μM, and more usually 0.5 nM to 200 nM. It is expected that serum concentrations of 0.5 nM to 20 nM should be sufficient in most circumstances.

To obtain these treatment concentrations, a patient in need of treatment likely will be administered between about 0.001 mg per day per kg of body weight and 50.0 mg per day per kg. Usually, not more than about 10.0 mg per day per kg of body weight of protein kinase C inhibitor should be needed. As noted above, the above amounts may vary on a case-by-case basis.

The effectiveness of the invention compounds on HIV related CNS diseases can be tested both *in vitro* and *in vivo*. See Toggas et al., Nature 367:188-193, 1994; and Wyss-Coray et al., J. Clin. Invest. 97:789-798, 1996 for detailed description. Both references are incorporated herein. The effects of the invention compounds on the phenotype of cultured glial cells overexpressing gp120 can be tested *in vitro*. An ability of the compounds to inhibit expression of the "activated astroglial cell phenotype" as exemplified by a reduction in expression of glial fibrillary acidic protein

(GFAP) would indicate a positive response of the compounds in attenuating CNS manifestations in HIV infected patients. Transgenic mice overexpressing gp120 can be used to test the effects of the invention compounds *in vivo*. Using histological analysis, compounds inducing a reduction in the reactive gliosis and the concomitant neurotoxicity in gp120 overexpressing mice would be highly predictive of beneficial effect of the compounds in treating the CNS diseases associated with HIV injection.

The compounds of formula I, and the preferred compounds of formula Ia and Ib are preferably formulated prior to administration. Suitable pharmaceutical formulations are prepared by known procedures using well known and readily available ingredients. In making the compositions suitable for use in the method of the present invention, the active ingredient will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders for either oral or topical application.

Some examples of suitable carriers, excipient, and diluents include lactose, dextrose, sucrose sorbitol, mannitol, starches, gum acacia, calcium phosphates, alignate, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetering agents or flavoring agents. The compositions of the invention may be fomulated so as to provide quick, sustained or delayed release of the active ingredient after administration to the patient. The compositions are preferably formulated in a unit dosage form,each dosage containing from about 0.05 mg to about 3 g, more usually about 750 mg of the active ingredient. However, it will be understood that the therapeutic dosage administered will be determined by the physician in the light of the relevant circumstances including the severity of the condition to be treated, the choice of compound to be administered and the chosen route of administration. Therefore, the above dosage ranges are not intended to limit the scope of the invention in any way. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

In addition to the above formulations, most of which may be administered orally, the compounds used in the method of the present invention also may be administered topically. Topical formulations include ointments, creams and gels.

Ointments generally are prepared using either (1) an oleaginous base, i.e., one consisting of fixed oils or hydrocarbons, such as white petrolatum or mineral oil, or (2) an absorbent base, i.e., one consisting of an anhydrous substance or substances which can absorb water, for example anhydrous lanolin. Customarily, following formation of the base, whether oleaginous or absorbent, the active ingredient (compound) is added to an amount affording the desired concentration.

Creams are oil/water emulsions. They consist of an oil phase (internal phase), comprising typically fixed oils, hydrocarbons, and the like, such as waxes, petrolatum, mineral oil, and the like, and an aqueous phase (continuous phase), comprising water and any water-soluble substances, such as added salts. The two phases are stabilized by use of an emulsifying agent, for example, a surface active agent, such as sodium lauryl sulfate; hydrophilic colloids, such as acacia colloidal clays, veegum, and the like. Upon formation of the emulsion, the active ingredient (compound) customarily is added in an amount to achieve the desired concentration.

Gels comprise a base selected from an oleaginous base, water, or an emulsion-suspension base. To the base is added a gelling agent which forms a matrix in the base, increasing its viscosity. Examples of gelling agents are hydroxypropyl cellulose, acrylic acid polymers, and the like. Customarily, the active ingredient (compounds) is added to the formulation at the desired concentration at a point preceding addition of the gelling agent.

The amount of compound incorporated into a topical formulation is not critical; the concentration should be within a range sufficient to permit ready application of the formulation to the affected tissue area in an amount which will deliver the desired amount of compound to the desired treatment site.

The customary amount of a topical formulation to be applied to on affected tissue will depend upon concentration of compound in the formulation. Generally, the formulation will be applied to the efected tissue in an amount affording from about 1 to about 500 $\mu g$ compound per $cm^2$ of an affected tissue. Preferably, the applied amount of compound will range from about 30 to about 300 $\mu g/cm^2$, more preferably, from about 50 to about 200 $\mu g/cm^2$, and, most preferably, from about 60 to about 100 $\mu g/cm^2$.

The following formulation examples are illustrative only and are not intended to limit the scope of the invention in any way.

Formulation 1

Hard gelatin capsules are prepared using the following ingredients:

| | Quantity (mg/capsule) |
|---|---|
| Active agent | 5 |
| starch, dried | 200 |
| magnesium stearate | 10 |
| Total | 215 mg |

The above ingredients are mixed and filled into hard gelatin capsules in 460 mg quantities.

Formulation 2

A tablet is prepared using the ingredients below.

| | Quantity (mg/capsule) |
|---|---|
| Active agent | 15 |
| cellulose, microcrystalline | 10 |
| silicon dioxide, fumed | 10 |
| stearic acid | 5 |
| Total | 40 mg |

The components are blended and compressed to form tablets each weighing 665 mg.

Formulation 3

Tablets each containing 60 mg of active ingredient are made as follows:

| | Quantity (mg/tablet) |
|---|---|
| Active agent | 60 mg |
| starch | 45 mg |
| microcrystalline cellulose | 35 mg |
| polyvinylpyrrolidone (as 10% solution in water) | 4 mg |
| sodium carboxymethyl starch | 4.5 mg |
| magnesium stearate | 0.5 mg |
| talc | 1 mg |
| Total | 150 mg |

The active ingredient, starch and cellulose are passed through a No. 43 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

The principles, preferred embodiments and modes of operation of the present invention have been described in the foregoing specification. The invention which is intended to be protected herein, however, is not to be construed as limited to the particular forms disclosed, since key are to be regarded as illustrative rather than restrictive. Variations and changes may be made by those skilled in the art without departing from the spirit of the invention.

## Claims

1. The use of an inhibitor of the β isozyme of protein kinase C in the preparation of a composition for the treatment of central nervous system disease associated with human immunodeficiency virus.

**2.** The use of claim 1 wherein the inhibitor of the β isozyme of protein kinase C is a bis-indolyimaleimide or a macrocyclic bis-indolylmaleimide.

**3.** The use of claim 1 or 2 wherein the inhibitor is isozyme selective and where the isozyme selectivity is selected from the group consisting of beta-1 and beta-2 isozymes.

**4.** The use of claim 1, 2 or 3 wherein the protein kinase C inhibitor has the following formula:

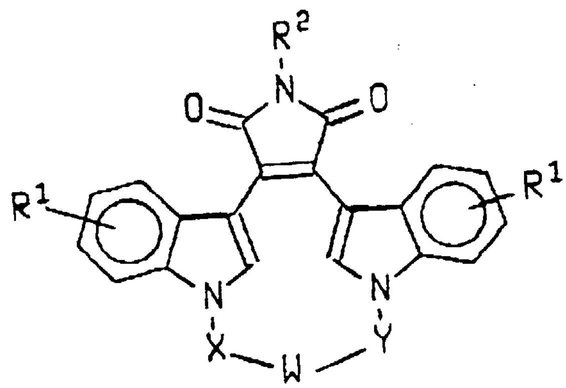

(I)

wherein:

W is O-, S-, SO-, -SO-2-, -CO-, $C_2$-$C_6$ alkylene, substituted alkylene, $C_2$-$C_6$ alkenylene, -aryl-, -aryl$(CH_2)_m$O-, -heterocycle-, -heterocycle-$(CH_2)_m$O-, -fused bicyclic-, -fused bicyclic-$(CH_2)_m$O-, -$NR^3$-, -$NOR^3$-, -CONH-, or -NHCO-;
X and Y are independently $C_1$-$C_4$ alkylene, substituted alkylene, or together X, Y, and W combine to form -$(CH_2)_n$-AA-;
$R^1$s are hydrogen or up to four optional substituents independently selected from halo, $C_1$-$C_4$ alkyl, hydroxy, $C_1$-$C_4$ alkoxy, haloalkyl, nitro, $NR^4R^5$, or -NHCO($C_1$-$C_4$ alkyl);
$R^2$ is hydrogen, $CH_3CO$-, $NH_2$, or hydroxy;
$R^3$ is hydrogen, $(CH_2)_m$aryl, $C_1$-$C_4$ alkyl, -COO($C_1$-$C_4$ alkyl), -$CONR^4R^5$, -(C=NH)$NH_2$, -SO($C_1$-$C_4$ alkyl), -$SO_2$($NR^4R^5$), or -$SO_2$ ($C_1$-$C_4$ alkyl);
$R^4$ and $R^5$ are independently hydrogen, $C_1$-$C_4$ alkyl, phenyl, benzyl, or combine to the nitrogen to which they are bonded to form a saturated or unsaturated 5 or 6 member ring;
AA is an amino acid residue;
m is independently 0, 1, 2, or 3; and
n is independently 2, 3, 4, or 5

or a pharmaceutically acceptable salt, prodrug or ester thereof.

**5.** The use of claim 4 wherein the protein kinase C inhibitor has the following formula:

(Ia)

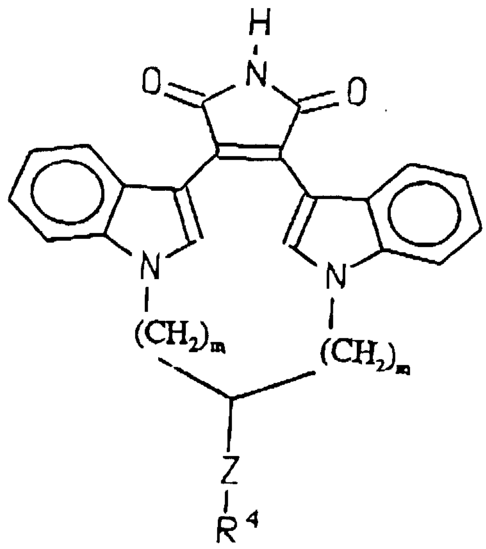

wherein Z is $-(CH_2)_p-$ or $-(CH_2)_p-O-(CH_2)_p-$; $R^4$ is hydroxy, -SH, $C_1$-$C_4$ alkyl, $(CH_2)_m$aryl, -NH(aryl), $-N(CH_3)(CF_3)$, $-NH(CF_3)$, or $-NR^5R^6$; $R^5$ is hydrogen or $C_1$-$C_4$ alkyl; $R^6$ is hydrogen, $C_1$-$C_4$ alkyl or benzyl; p is 0, 1, or 2; and m is independently 2 or 3, or a pharmaceutically acceptable salt, prodrug or ester thereof.

6. The use of claim 4 wherein the protein kinase C inhibitor has the following formula:

(Ib)

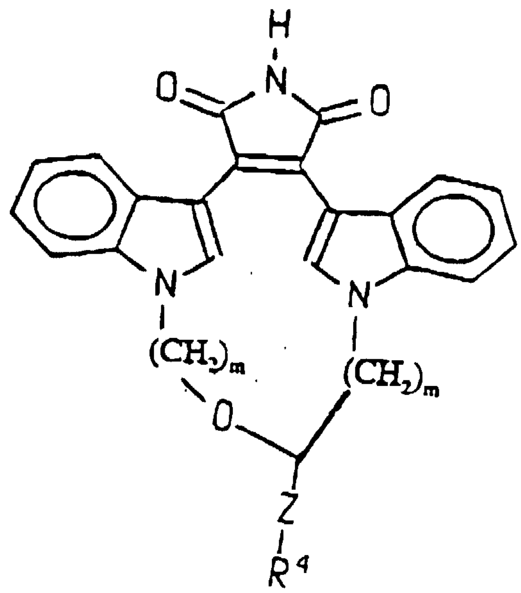

wherein Z is $-(CH_2)_p-$; $R^4$ is $-NR^5R^6$, $-NH(CF_3)$, or $-N(CH_3)(CF_3)$; $R^5$ and $R^6$ are independently H or $C_1$-$C_4$ alkyl; p is 0, 1, or 2; and m is independently 2 or 3, or a pharmaceutically acceptable salt, prodrug or ester thereof.

7. The use of claim 4, wherein the protein kinase C inhibitor comprises (S)-3,4-[N, N'-1,1'-((2"-ethoxy)-3'"(O)-4'"-(N, N-dimethylamino)-butane)-bis-(3,3'-indolyl)]-1(H)-pyrrole-2,5-dione or its pharmaceutically acceptable acid salt.

8. A use of claim 7, wherein the pharmaceutically acceptable acid salt is selected from the hydrochloride salt and the mesylate salt.

9. The use of an inhibitor of the β isozyme of protein kinase C in the preparation of a pharmaceutical composition for the treatment of or inhibition of gp 120 induced central nervous system disease.

**10.** The use of claim 9 wherein the inhibitor of the β isozyme of protein kinase C is a bis-indolyimaleimide or a macrocyclic bis-indolylmaleimide.

**11.** The use of claim 9 or 10 wherein the inhibitor is isozyme selective and where the isozyme selectivity is selected from the group consisting of beta-1 and beta-2 isozymes.

**12.** The use of claim 9, 10 or 11 wherein the protein kinase C inhibitor has the following formula:

(I)

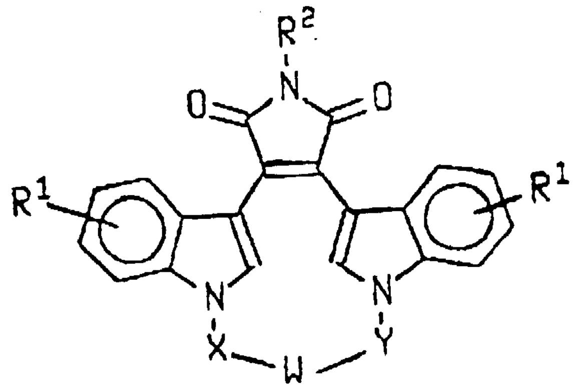

wherein:

W is -O-, -S-, -SO-, $-SO_2-$, -CO-, $C_2$-$C_6$ alkylene, substituted alkylene, $C_2$-$C_6$ alkenylene, -aryl-, $-aryl)CH_2)_mO-$, -heterocycle-, $-heterocycle-(CH_2)_mO-$, -fused bicyclic-, $-fused bicyclic-(CH_2)_mO-$, $-NR_3-$, $-NOR_3-$, -CONH-, or -NHCO-;
X and Y are independently $C_1$-$C_4$ alkylene, substituted alkylene, or together X, Y, and W combine to form $-(CH_2)_n-AA-$;
$R^1$s are hydrogen or up to four optional substituents independently selected from halo, $C_1$-$C_4$ alkyl hydroxy, $C_1$-$C_4$ alkoxy, haloalkyl, nitro, $NR^4R^5$, or $-NHCO(C_1$-$C_4$ alkyl);
$R^2$ is hydrogen, $CH_3CO-$, $NH_2$, or hydroxy;
$R^3$ is hydrogen, $(CH_2)_m$aryl, $C_1$-$C_4$ alkyl, $-COO(C_1$-$C_4$ alkyl), $-CONR^4R^5$,$-(C{=}NH)NH_2$, $-SO(C_1$-$C_4$ alkyl), $-SO_2(NR^4R^5)$, or $-SO_2$ $(C_1$-$C_4$ alkyl);
$R^4$ and $R^5$ are independently hydrogen, $C_1$-$C_4$ alkyl, phenyl, benzyl, or combine to the nitrogen to which they are bonded to form a saturated or unsaturated 5 or 6 member ring;
AA is an amino acid residue;
m is independently 0, 1, 2, or 3; and
n is independently 2, 3, 4, or 5

or a pharmaceutically acceptable salt, prodrug or ester thereof.

**13.** The use of claim 12 wherein the protein kinase C inhibitor has the following formula:

(Ia)

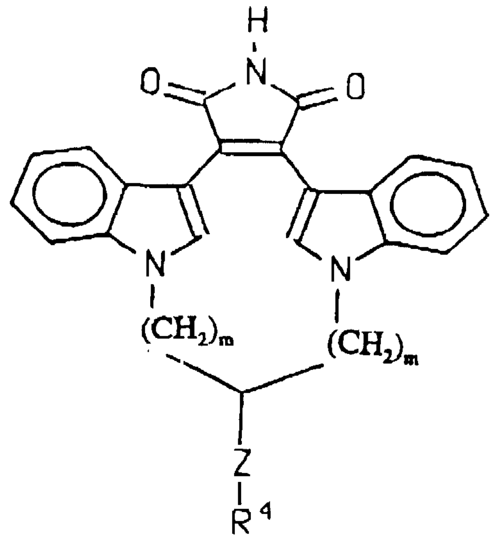

wherein Z is $-(CH_2)_p-$ or $-(CH_2)_p-O-(CH_2)_p-$; $R^4$ is hydroxy, -SH, $C_1$-$C_4$ alkyl, $(CH_2)_m$aryl, -NH(aryl), $-N(CH_3)(CF_3)$, $-NH(CF_3)$, or $-NR^5R^6$; $R^5$ is hydrogen or $C_1$-$C_4$ alkyl; $R^6$ is hydrogen, $C_1$-$C_4$ alkyl or benzyl; p is 0, 1, or 2; and m is independently 2 or 3, or a pharmaceutically acceptable salt, prodrug or ester thereof.

14. The use of claim 12 wherein the protein kinase C inhibitor has the following formula:

(Ib)

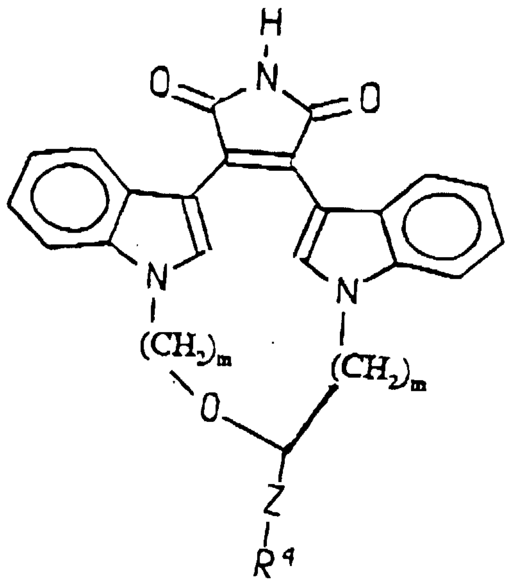

wherein Z is $(CH_2)_p-$; $R^4$ is $-NR^5R^6$, $-NH(CF_3)$, or $-N(CH_3)(CF_3)$; $R^5$ and $R^6$ are independently H or $C_1C_4$ alkyl; p is 0, 1, or 2; and m is independently 2 or 3, or a pharmaceutically acceptable salt, prodrug or ester thereof.

15. The use of claim 12, wherein the protein kinase C inhibitor comprises (S)-3,4-[N, N'-1,1'-((2''-ethoxy)-3'''(O)-4'''-(N,N-dimethylamino)-butane)-bis-(3,3'-indolyl)]-1(H)-pyrrole-2,5-dione or its pharmaceutically acceptable acid salt.

16. A use of claim 15, wherein the pharmaceutically acceptable acid salt is selected from the hydrochloride salt and the mesylate salt.

European Patent Office

# EUROPEAN SEARCH REPORT

Application Number
EP 97 30 6716

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X<br>Y | WO 95 00520 A (CIBA-GEIGY AG)<br>* abstract *<br><br>* page 3, paragraph 3 *<br>* page 5, paragraph 2 *<br>--- | 1-3,9-11<br>4-10,<br>12-16 | A61K31/40 |
| Y | US 5 506 231 A (STUART A. LIPTON)<br>* column 1 - column 2 *<br>--- | 1-16 | |
| D,Y | EP 0 657 458 A (ELI LILLY AND COMPANY)<br>* page 3, line 58 - page 5, line 13 *<br>* example 5S *<br>* page 6, line 28 - page 8, line 36 *<br>* page 11, line 33 - line 40 *<br>--- | 1-16 | |
| Y | T.WISS-CORAY ET AL.: "Dysregulation of signal transduction pathways as a potentail mechanism of nervous system alterations in HIV-1 gp120 transgenic mice and humans with HIV-1 encephalitis"<br>J.CLIN.INVEST.,<br>vol. 97, no. 3, February 1996,<br>pages 789-798, XP002052501<br>* abstract *<br>* page 797 *<br>--- | 1-16 | TECHNICAL FIELDS SEARCHED (Int.Cl.6)<br>A61K |
| Y | PROTUL SHRIKANT ET AL.: "HIV glycoprotein 120 enhances intercellular adhesion molecule-1 gene expression in glial cells"<br>J.IMMUNOL.,<br>vol. 156, no. 3, February 1996,<br>pages 1307-1314, XP002052502<br>* abstract *<br>---<br>-/-- | 1-16 | |

The present search report has been drawn up for all claims

| Place of search | Date of completion of the search | Examiner |
|---|---|---|
| MUNICH | 19 January 1998 | Tzschoppe, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 97 30 6716

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.6) |
| Y | NEREIDA A. PARADA ET AL.: "IL-16 and other CD4 ligand induced migration is dependent upon protein kinase C" CELL.IMMUNOL., vol. 168, no. 1, February 1996, pages 100-106, XP002052503 * abstract * ----- | 1-16 | |
| | | | TECHNICAL FIELDS SEARCHED (Int.Cl.6) |
| | The present search report has been drawn up for all claims | | |

| Place of search | Date of completion of the search | Examiner |
|---|---|---|
| MUNICH | 19 January 1998 | Tzschoppe, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)